# EUROPEAN PATENT APPLICATION

(11) **EP 4 122 450 A1**
(43) Date of publication of application: **25.01.2023**
(21) Application number: 22275095.2
(22) Date of filing: 11.07.2022
(51) Int. Cl.: A61K 9/08, A61K 31/4168, A61K 9/00, A61K 47/10, A61K 47/26

(54) **LIQUID PHARMACEUTICAL COMPOSITION OF CLONIDINE**

(30) Priority: 20.07.2021 GB 202110420
(71) Applicant: Rosemont Pharmaceuticals Ltd, Leeds LS11 9XE (GB)
(72) Inventor: Carr, Scott, Leeds, LS11 9XE (GB); Mlambo, Khonzanani, Leeds, LS11 9XE (GB)
(74) Representative: Tomkinson, Alexandra

(57) **Abstract**

There is provided a liquid pharmaceutical composition suitable for oral administration comprising clonidine or a pharmaceutically acceptable salt thereof and at least one carrier agent to dissolve or substantially dissolve the clonidine or the pharmaceutically acceptable salt of clonidine therein. The pharmaceutical composition does not contain a buffer.

## Description

The present invention relates to liquid pharmaceutical compositions of Clonidine or pharmaceutically acceptable salts thereof suitable for oral administration.

Clonidine is an imidazole derivative that acts as an alpha-2 adrenergic agonist used to treat disorders, such as for example, hypertension, migraine headaches, prevention of recurrent migraines, prevention of vascular headaches, menopausal symptoms - particularly flushing and vasomotor conditions [1], Attention Deficit Hyperactivity Disorder (ADHD) [2] and severe pain. It is also used to treat withdrawal symptoms from various drug dependencies. The chemical name for Clonidine is N-(2,6-dichlorophenyl)-4,5-dihydro-1H-imidazol-2-amine and the structure of the same is shown below.

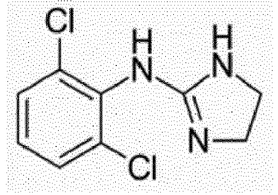

A current commercially available clonidine product is in the form of a hydrochloride salt and is sold under the brand name Catapres^{®}. It is a tablet form suitable for oral administration in three dosage strengths of 0.1mg, 0.2mg and 0.3mg. However, a problem with this solid tablet form of medication is that it cannot be used by patients who have difficulties swallowing, such as elderly people, disabled people, children and patients with certain conditions.

In an attempt to overcome the abovementioned problems, it is known to provide oral liquid formulations of Clonidine. An example of one such oral liquid formulation is disclosed in WO2018/002751 which discloses a liquid pharmaceutical composition for oral administration comprising Clonidine or pharmaceutically acceptable salts thereof present in the range of 0.0005 to about 0.002% w/v. The composition includes at least one buffer provided at a buffer strength in the range of 5-25 millimolar to maintain the pH of the composition at between 4-7 for stability purposes, and at least one preservative. The Clonidine or pharmaceutically acceptable salt thereof is present in the composition in the range from 5mcg/ml to 20mcg/ml.

It is an aim of the present invention to provide an alternative liquid pharmaceutical composition of Clonidine suitable for oral administration.

It is a further aim of the present invention to provide a method of use and/or manufacture of an alternative liquid pharmaceutical composition of Clonidine suitable for oral administration.

According to a first aspect of the present invention there is provided a liquid pharmaceutical composition suitable for oral administration comprising Clonidine or a pharmaceutically acceptable salt thereof, at least one carrier agent to dissolve or substantially dissolve the Clonidine or the pharmaceutically acceptable salt of Clonidine therein, and wherein the composition does not contain a buffer.

The Applicants have surprisingly found that a liquid pharmaceutical composition of Clonidine or a pharmaceutically acceptable salt thereof can be provided which is stable and self-preserving without the use of a buffer or buffer agent. This is surprising and unexpected.

Preferably the pharmaceutically acceptable salt of Clonidine used in the composition is Clonidine hydrochloride.

Preferably the at least one carrier agent is, includes or consists of purified water and/or an alcohol.

In one example the alcohol is ethanol.

Preferably the purified water and/or alcohol is present in an amount of between 0-10% w/v, at least about 10% w/v, and further preferably at least about 70% w/v in one example, and yet further preferably at least about 80% w/v in one example.

In one embodiment purified water is present in an amount of at least or equal to about 37% w/v, and further preferably in an amount of at least or equal to about 63% w/v, and yet further preferably in an amount of at least or equal to about 70%, and yet further preferably in an amount of at least or equal to about 80%.

In one embodiment the composition contains at least one preservative or preservative agent.

Preferably the at least one preservative or preservative agent is any or any combination of methyl parahydroxybenzoate (methyl paraben), propyl parahydroxybenzoate (propyl paraben), methyl hydroxybenzoate, propyl hydroxybenzoate, ethyl paraben, butyl paraben (free base and salts thereof), benzoic acid (free base and salts thereof), bronopol, imidurea, potassium sorbate, phenoxyethanol, phenylmercuric acetate, benzyl alcohol, phenylmercuric borate, Chlorocresol, Benzethonium Chloride, Phenylethyl Alcohol, Benzalkonium Chloride, Methylparaben, Hexetidine, Chlorobutanol, Ethylparaben, Propylparaben, Sodium Benzoate, Potassium Benzoate, Sorbic Acid, Cresol, Propylparaben Sodium, Cetylpyridinium Chloride, Phenylmercuric Nitrate, Chloroxylenol, Propionic Acid, Phenol, Thimerosal, Sulfur Dioxide, Boric Acid, Edetic Acid, Sodium Propionate, Calcium Chloride, Sodium Acetate, Sodium Sulfite, Benzoic Acid, Monothioglycerol, Cetrimide, Calcium Acetate, Butylene Glycol, Sodium Metabisulfite, Alcohol, Propyl Gallate, Potassium Metabisulfite, Sodium Lactate, Chlorhexidine, Calcium Lactate, Pentetic Acid, Glycerin, Propylene Glycol Alginate, Sodium Borate, Magnesium Trisilicate, Isopropyl Alcohol, Dimethyl Ether, Propylene Glycol, Butylated Hydroxyanisole, Pyrrolidone, Lactic Acid, Sodium Lauryl Sulfate and Dimethyl Sulfoxide and/or the like.

Preferably the at least one preservative or preservative agent is present in an amount of about 0.001 % w/v to about 0.3% w/v, and further preferably is an amount of above 0.002% to about 0.21%.

Preferably the at least one preservative or preservative agent includes or consists of about 0.001%-0.31% w/v of methyl paraben or methyl hydroxybenzoate, and further preferably about 0.206% w/v of methyl paraben or methyl hydroxybenzoate.

Preferably the at least one preservative or preservative agent includes about 0.01%-0.03% w/v of propyl paraben or propyl hydroxybenzoate, and further preferably about 0.0224% w/v of propyl paraben or propyl hydroxybenzoate.

In one embodiment the composition includes about 0.006%-0.05% w/v of butyl paraben.

In one embodiment the preservative or preservative agent in the composition consists of methyl paraben or methyl hydroxybenzoate, and propyl paraben or propyl hydroxybenzoate.

In one embodiment the preservative or preservative agent in the composition consists of 0.206% w/v of methyl paraben or methyl hydroxybenzoate, and 0.0224% w/v of propyl paraben or propyl hydroxybenzoate.

In one embodiment the composition does not contain a preservative or preservative agent in addition to not containing a buffer or buffer agent. Thus, the composition is stable and self-preserving. This is also surprising and unexpected.

In the embodiment, the at least one carrier agent is, includes or consists of any or any combination of purified water, ethanol, acetone, alcohol, benzyl alcohol, benzyl benzoate, butylene glycol, dibutyl phthalate, diethyl phthalate, dimethyl phthalate, dimethyl sulfoxide, dimethylacetamide, glycofurol, glycerin, isopropyl alcohol, isopropyl myristate, isopropyl palmitate, polyethylene glycol, propylene carbonate, pyrrolidone, triacetin, triethyl citrate, triolein, glycerol, sorbitol or liquid mannitol.

In one embodiment the at least one carrier agent, the preservative and/or the Clonidine or a pharmaceutically acceptable salt thereof is heated to help dissolve the same during mixing.

Preferably the at least one carrier agent, the preservative and/or the Clonidine or a pharmaceutically acceptable salt thereof is heated is between 65-75 °C.

In one embodiment the composition includes a sweetener or at least one sweetener agent. For example, the sweetener or at least one sweetener agent can include sucralose, Advantame, Acesulfame potassium, Neotame, saccharin, aspartame, neohesperidin, sucralose, stevia, glycerol, sorbitol, mannitol, xylitol, and/or liquid maltitol.

Preferably the sweetener or at least one sweetener agent is used in an amount of about 0.1%-0.5% w/v, and further preferably is used in an amount of about 0.20% 0.25% w/v.

In one example, 100% neat glycerol could be used as a sweetener agent and as a carrier agent.

In one embodiment the clonidine or pharmaceutically acceptable salt thereof is present in an amount of about 0.0005 to about 0.004% w/v.

In one embodiment the clonidine or pharmaceutically acceptable salt thereof is present in an amount of about 0.001% w/v.

Preferably the composition is a liquid oral solution composition.

The term "about" in the present application means +/-10% of the mentioned value.

Preferably the composition does not contain any anti-oxidants or anti-oxidant agents.

Preferably the composition does not contain any colourants or colourant agents.

According to one aspect of the present invention there is a provided a method of manufacturing a liquid pharmaceutical composition suitable for oral administration comprising Clonidine or a pharmaceutically acceptable salt thereof, said method including the steps of dissolving or substantially dissolving the Clonidine or the pharmaceutically acceptable salt of Clonidine in at least one carrier agent, and wherein the composition does not contain a buffer.

In one embodiment the method includes the step of adding at least one preservative or preservative agent.

In one embodiment the method the composition does not contain a preservative or preservative agent in addition to not containing a buffer or buffer agent.

According to one aspect of the present invention, there is provided a liquid pharmaceutical composition suitable for oral administration comprising or consisting of Clonidine or a pharmaceutically acceptable salt thereof, purified water and glycerol.

Preferably the Clonidine or a pharmaceutically acceptable salt thereof is present in in an amount of 0.001% w/v.

Preferably the purified water is present in an amount of about 10% w/v.

Preferably the glycerol is present in an amount taking the w/v of the composition between about 27%-99%, further preferably at least about 40%, and further preferably at least about 63%, and preferably in an amount taking the composition to about 100% once the other components of the composition have been added.

According to a further aspect of the present invention, there is provided a liquid pharmaceutical composition suitable for oral administration comprising or consisting of Clonidine or a pharmaceutically acceptable salt thereof, at least one preservative, at least one sweetener, and purified water.

Preferably the Clonidine or a pharmaceutically acceptable salt thereof is present in an amount of about 0.001% w/v.

In one embodiment the preservative or preservative agent in the composition consists of methyl paraben or methyl hydroxybenzoate, and propyl paraben or propyl hydroxybenzoate.

In one embodiment the preservative or preservative agent in the composition consists of 0.206% w/v of methyl paraben or methyl hydroxybenzoate, and 0.0224% w/v of propyl paraben or propyl hydroxybenzoate.

Preferably the at least one preservative is present in an amount of about 0.2284% w/v.

Preferably the at least one sweetener is present in an amount of about 0.2% w/v, 0.25% w/v between 0.02- 0.25% w/v.

Preferably the purified water is present in an amount of at least about 70%, preferably at least about 80%, and preferably in an amount taking the composition to about 100%.

In one embodiment the dosage of clonidine in the liquid composition is in the range of 2mcg/ml-500mcg/ml. Further preferably 50mcg/5ml or 100mcg/5ml.

Preferably the composition of the present invention is administered in a dosage of 50mcg-100mcg up to three times daily.

In one embodiment the liquid pharmaceutical composition of the present invention can be used in the treatment of any or any combination of hypertension, attention deficit hyperactivity disorder, drug withdrawal symptoms, pain conditions, menopausal flushing, migraine headaches and/or the like.

Preferably the composition is a liquid oral solution at room temperature (i.e. approximately 21 degrees Celsius) and pressure (i.e. approximately 1 atmosphere or 1013hpa).

The present invention will now be illustrated by way of the following examples.

### Example 1

A liquid oral solution pharmaceutical composition of clonidine hydrochloride - 50mcg/5ml is prepared which contains no buffers, and/or is paraben free, yet remains stable for a minimum period of time. The formulation is self-preserving as the glycerol bulking agent is acting as an antimicrobial agent. In Pharmaceutical Oral Solutions, glycerol is used as a solvent, sweetening agent, antimicrobial preservative and viscosity increasing agent.

The formulation is shown in Table 1 below.

**Table 1**

| **Clonidine Hydrochloride 50mcg/5ml Oral Solution** | | |
|---|---|---|
| Component | % w/v | Quantity required |
| Glycerol (A) | 40% | 400.0g |
| Purified water (B) | 10% | 100.0g |
| Clonidine Hydrochloride (C) | 0.001% | 0.01g |
| Glycerol (D) | To 100% | To 1000ml |

The Clonidine hydrochloride is first dissolved in the purified water. The purified water with dissolved clonidine hydrochloride is then added to the glycerol until the required amount is reached and mixed until uniform or substantially uniform with a propellor mixer at room temperature and pressure.

**Preservative Efficacy Results for the Composition in Table 1 and similar formulations with different levels of glycerol (63%-90% w/v):**

| **Organism** | | Log Values | | | Log Reduction | |
|---|---|---|---|---|---|---|
| | | 0 | 14 Day | 28 Day | 0-14 Day | 14 - 28 Day |
| PET 15 D557-1/032A | ***E. coli*** | 7.72 | <2.00 | <1.00 | >-5.72 | >-1.00 |
| | ***P***. ***aeruginosa*** | 7.68 | <2.00 | <1.00 | >-5.68 | >-1.00 |
| | ***S**. **aureus*** | 7.60 | <2.00 | <1.00 | >-5.60 | >-1.00 |
| 3149.956mg/ 5ml Glycerol | ***A**. **brasiliensis*** | 6.67 | 4.46 | 3.81 | -2.21 | -0.65 |
| | ***C**. **albicans*** | 7.77 | <3.00 | <1.00 | >-4.77 | >-2.00 |
| | ***Z**. **rouxii*** | 7.72 | <3.00 | 3.27 | >-4.72 | <+0.27 |

| **Organism** | | Log Values | | | Log Reduction | |
|---|---|---|---|---|---|---|
| | | 0 | 14 Day | 28 Day | 0-14 Day | 14 - 28 Day |
| PET 16 D557-1/032B 3599.96mg/5 ml Glycerol | ***E**. **coli*** | 7.72 | <2.00 | <1.00 | >-5.72 | >-1.00 |
| | ***P***. ***aeruginosa*** | 7.68 | <2.00 | <1.00 | >-5.68 | >-1.00 |
| | ***S**. **aureus*** | 7.60 | <2.00 | <1.00 | >-5.60 | >-1.00 |
| | ***A**. **brasiliensis*** | 6.67 | <3.00 | <1.00 | >-3.67 | >-2.00 |
| | ***C**. **albicans*** | 7.77 | <3.00 | <1.00 | >-4.77 | >-2.00 |
| | ***Z**. **rouxii*** | 7.72 | <3.00 | <1.00 | >-4.72 | >-2.00 |

| **Organism** | | Log Values | | | Log Reduction | |
|---|---|---|---|---|---|---|
| | | 0 | 14 Day | 28 Day | 0-14 Day | 14 - 28 Day |
| PET 17 D557-1/032C 4049.955mg/ 5ml Glycerol | ***E**. **coli*** | 7.72 | <2.00 | <1.00 | >-5.72 | >-1.00 |
| | ***P***. ***aeruginosa*** | 7.68 | <2.00 | <1.00 | >-5.68 | >-1.00 |
| | ***S**. **aureus*** | 7.60 | <2.00 | <1.00 | >-5.60 | >-1.00 |
| | ***A**. **brasiliensis*** | 6.67 | <3.00 | <1.00 | >-3.67 | >-2.00 |
| | ***C**. **albicans*** | 7.77 | <3.00 | <1.00 | >-4.77 | >-2.00 |
| | ***Z.rouxii*** | 7.72 | <3.00 | <1.00 | >-4.72 | >-2.00 |

| **Organism** | | Log Values | | | Log Reduction | |
|---|---|---|---|---|---|---|
| | | 0 | 14 Day | 28 Day | 0-14Day | 14-28Day |
| PET 18 | ***E**. **coli*** | 7.72 | <2.00 | <1.00 | >-5.72 | >-1.00 |
| D557-1/032D 4499.95mg/5 ml Glycerol | ***P***. ***aeruginosa*** | 7.68 | <2.00 | <1.00 | >-5.68 | >-1.00 |
| | ***S**. **aureus*** | 7.60 | <2.00 | <1.00 | >-5.60 | >-1.00 |
| | ***A**. **brasiliensis*** | 6.67 | <3.00 | <1.00 | >-3.67 | >-2.00 |
| | ***C**. **albicans*** | 7.77 | <3.00 | <1.00 | >-4.77 | >-2.00 |
| | ***Z**. **rouxii*** | 7.72 | <3.00 | <1.00 | >-4.72 | >-2.00 |

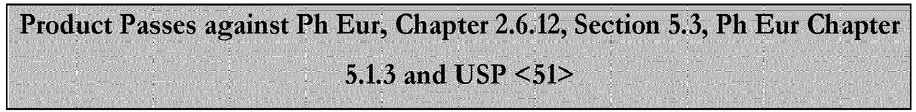

### Recommended Efficacy (Ph. Eur):

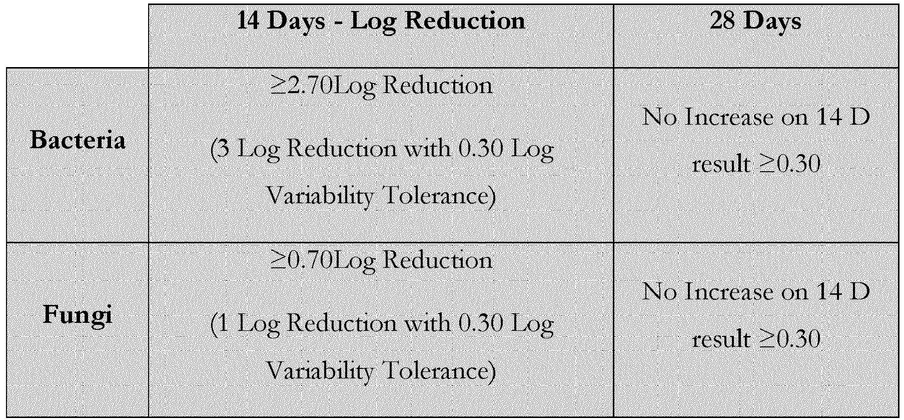

### Example 2

A liquid oral solution pharmaceutical composition of clonidine hydrochloride - 50mcg/5ml is prepared which contains no buffers but does contain preservatives, yet remains stable for a minimum period of time.

The formulation is shown in Table 2 below.

**Table 2**

| **Clonidine Hydrochloride 50mcg/5ml Oral Solution** | | |
|---|---|---|
| **Component** | **%w/v** | **Quantity required** |
| Purified water (A) | 35% | 350.0g |
| Purified water (B) | 10% | 350.0g |
| Methyl Paraben | 0.206% | 2.06g |
| Propyl Paraben | 0.0224% | 0.224g |
| Clonidine HCl | 0.001% | 0.01g |
| Sucralose | 0.25% | 2.50g |
| Purified water (C) | 25% | To 1000ml |
| Purified Water (D) | Make up to 100% | |

Purified water (A) is first added to the main vessel.

To a separate stage vessel 1, the Clonidine hydrochloride is dissolved in the purified water (B).

Separate stage 1 vessel content is then added to the main vessel and mixed.

To another separate stage vessel 2, purified water (c) is added and is heated to 60-70 degrees Celsius.

The preservatives methyl paraben and propyl paraben are mixed in vessel 2 until uniform or substantially uniform with a propellor mixer. The mixture is cooled to room temperature.

Separate stage 2 vessel content is then added to the main vessel and mixed.

The sucralose is then added to the main vessel and mixed.

The final volume of the composition is made is made up to the required amount using purified water.

The formulations of the present invention are expected to be stable for at least approximately two years. Tests undertaken to check the stability are HPLC assay testing for the API and degradation products, HPLC assay testing for preservatives, microbial testing and physical testing (i.e. looking at density, pH, appearance, viscosity and/or the like).

### Example 3

A further example of a liquid oral solution pharmaceutical composition of clonidine hydrochloride - 10mcg/1ml is prepared which contains no buffers but does contain preservatives, yet remains stable for a minimum period of time.

The formulation is shown in Table 3 below.

**Table 3**

| **Clonidine Hydrochloride 10mcg/1ml Oral Solution** | | |
|---|---|---|
| **Component** | **%w/v** | **Quantity required** |
| Purified water (A) | 55% | 550.00mg |
| Methyl Hydroxybenzoate (B) | 0.206% | 2.06mg |
| Propyl Hydroxybenzoate (C) | 0.0224% | 0.224mg |
| Purified Water (D) | 25% | 250.00mg |
| Sucralose (E) | 0.2% | 2.00mg |
| Purified Water (F) | 0.2% | 2.00mg |
| Clonidine Hydrochloride (G) | 0.001% | 10.00mcg |
| Purified Water (H) | Make up to 100% | Make up to 1ml |

1. Purified water (A) is first added to the main vessel and is heated up to 75-85 °C.
2. The preservatives: Methyl Hydroxybenzoate (B), followed by Propyl Hydroxybenzoate (C) are added to the main vessel and mixed with a mixing device (such as for example a propeller mixer) until fully dissolved. The temperature is maintained between 75-85 °C.
3. Purified Water (D) is then added to the main vessel to cool down the contents to 65 °C and the mixture is mixed with a mixing device (such as for example a propeller mixer) until homogenous or substantially homogenous.
4. Sucralose (E) is then added to the main vessel and mixed until dissolved.
5. Purified Water (F) is added to a separate vessel.
6. The Clonidine hydrochloride is added into the separate vessel and dissolved completely in the purified water (F).
7. The contents of the separate vessel are then added to the main vessel and mixed with a mixing device until homogenous or substantially homogenous. The separate vessel can be rinsed after the transfer with purified water and all washings transferred to the main vessel.
8. Purified water (H) is then added to the main vessel to make a final volume and mixed with a mixing device until homogenous or substantially homogenous.
9. Check the pH is within range (4-7). The pH is not adjusted but is normally approximate pH 5.0.
10. Fill 103 +/-2ml of the finished product into 100ml amber glass bottles and close with child resistant closure.

Stability Data was obtained for the above formulation and is shown in Table 4 below.

### Table 4 Assay of Clonidine Hydrochloride after storage over time at different temperatures

The stability data is a % of the Clonidine Hcl in solution. A reduction of the % of Clonidine Hcl over time reflect instability of the solution. It can be seen from the data below that the solution is very stable

| Clonidine HCl 50mcg/5ml Oral Solution | | | | | |
|---|---|---|---|---|---|
| Ref:D557_DS03 | | Clonidine Hydrochloride Assay % label Claim | | | |
| | Months | | | | |
| Condition of Storage | 0 | 1 | 3 | 6 | 9 |
| 5°C | 108.15 | | 108.43 | | |
| 25°C | 108.15 | | 108.33 | 108.54 | 109.79 |
| 30°C | 108.15 | | 108.36 | 108.49 | 109.23 |
| 40°C | 108.15 | 106.34 | 108.48 | | |
| 50°C | 108.15 | 106.57 | 108.59 | | |

Table foot notes:
1 No API related degradants are detected at 9 months i.e. <0.1% (reporting threshold).
2 No API related degradants are seen, the Limit of Detection of the method is about 0.02%.

### References

[1]. (https://bnf.nice.org.uk/drug/clonidine-hydrochloride.html)
[2] - "Clonidine for treating attention deficit hyperactivity disorder in children and young people" - National Institute for Health & Care Excellence - 2nd April 2013 - nice.org.uk.

## Claims

1. A liquid pharmaceutical composition suitable for oral administration comprising clonidine or a pharmaceutically acceptable salt thereof, at least one carrier agent to dissolve or substantially dissolve the clonidine or the pharmaceutically acceptable salt of clonidine therein, and wherein the pharmaceutical composition does not contain a buffer.

2. The liquid pharmaceutical composition according to claim 1, wherein the pharmaceutically salt used in the composition is clonidine hydrochloride.

3. The liquid pharmaceutical composition according to claims 1 or 2, wherein the at least one carrier agent is, includes or consists of any or any combination of purified water, ethanol, acetone, an alcohol, benzyl alcohol, benzyl benzoate, butylene glycol, dibutyl phthalate, diethyl phthalate, dimethyl phthalate, dimethyl sulfoxide, dimethylacetamide, glycofurol, glycerin, isopropyl alcohol, isopropyl myristate, isopropyl palmitate, polyethylene glycol, propylene carbonate, pyrrolidone, triacetin, triethyl citrate, triolein, glycerol, sorbitol or liquid mannitol.

4. The liquid pharmaceutical composition according to claim 3, wherein the purified water and/or the alcohol is used in an amount of between 0-10% w/v, at least about 10% w/v, at least about 70% w/v or at least about 80% w/v.

5. The liquid pharmaceutical composition according to any preceding claim, wherein the composition includes at least one preservative or preservative agent.

6. The liquid pharmaceutical composition according to claim 5, wherein the at least one preservative or preservative agent is any or any combination of methyl parahydroxybenzoate (methyl paraben), methyl hydroxybenzoate, propyl hydroxybenzoate, propyl parahydroxybenzoate (propyl paraben), ethyl paraben, butyl paraben (free base and salts thereof), benzoic acid (free base and salts thereof), bronopol, imidurea, potassium sorbate, phenoxyethanol, phenylmercuric acetate, benzyl alcohol, phenylmercuric borate, Chlorocresol, Benzethonium Chloride, Phenylethyl Alcohol, Benzalkonium Chloride, Methylparaben, Hexetidine, Chlorobutanol, Ethylparaben, Propylparaben, Sodium Benzoate, Potassium Benzoate, Sorbic Acid, Cresol, Propylparaben Sodium, Cetylpyridinium Chloride, Phenylmercuric Nitrate, Chloroxylenol, Propionic Acid, Phenol, Thimerosal, Sulfur Dioxide, Boric Acid, Edetic Acid, Sodium Propionate, Calcium Chloride, Sodium Acetate, Sodium Sulfite, Benzoic Acid, Monothioglycerol, Cetrimide, Calcium Acetate, Butylene Glycol, Sodium Metabisulfite, Alcohol, Propyl Gallate, Potassium Metabisulfite, Sodium Lactate, Chlorhexidine, Calcium Lactate, Pentetic Acid, Glycerin, Propylene Glycol Alginate, Sodium Borate, Magnesium Trisilicate, Isopropyl Alcohol, Dimethyl Ether, Propylene Glycol, Butylated Hydroxyanisole, Pyrrolidone, Lactic Acid, Sodium Lauryl Sulfate and Dimethyl Sulfoxide.

7. The liquid pharmaceutical composition according to claims 5 or 6, wherein the at least one preservative or preservative agent is present in the composition in an amount of about 0.001% w/v to about 0.3% w/v.

8. The liquid pharmaceutical composition according to any of claims 5-7, wherein the at least one preservative or preservative agent includes about 0.015%-0.310% w/v of methyl paraben or methyl hydroxybenzoate and/or includes about 0.01%-0.03% w/v of propyl paraben or propyl hydroxybenzoate.

9. The liquid pharmaceutical composition according to claim 1, wherein the composition does not contain a preservative or preservative agent, an anti-oxidant and/or colourants.

10. The liquid pharmaceutical composition according to any preceding claim, wherein the composition includes a sweetener or at least one sweetener agent.

11. The liquid pharmaceutical composition according to claim 16, wherein the sweetener or at least one sweetener agent includes sucralose, Advantame, Acesulfame potassium, Neotame, saccharin, aspartame, neohesperidin, sucralose, stevia, glycerol, sorbitol, mannitol, xylitol, and/or liquid maltitol.

12. The liquid pharmaceutical composition according to claims 16 or 17, wherein the sweetener or sweetener agent is used in an amount of about 0.1%-0.5% w/v.

13. The liquid pharmaceutical composition according to any preceding claim, wherein the clonidine or pharmaceutically acceptable salt thereof is present in an amount of about 0.0005% to about 0.004% w/v.

14. The liquid pharmaceutical composition according to any preceding claim, wherein the composition consists of glycerol, purified water and clonidine or a pharmaceutically acceptable salt of clonidine.

15. A method of manufacturing a liquid pharmaceutical composition suitable for oral administration comprising clonidine or a pharmaceutically acceptable salt thereof, said method including the steps of dissolving or substantially dissolving the clonidine or the pharmaceutically acceptable salt of clonidine in at least one carrier agent, and wherein the pharmaceutical composition does not contain a buffer.
